# EUROPEAN PATENT APPLICATION

(11) **EP 4 542 558 A1**
(43) Date of publication of application: **23.04.2025**
(21) Application number: 23203707.7
(22) Date of filing: 16.10.2023
(51) Int. Cl.: G16H 20/10, G16H 50/20

(54) **METHOD FOR DETERMINING EFFICACY OF A MEDICAL TREATMENT**

(71) Applicant: Koninklijke Philips N.V., 5656 AG Eindhoven (NL)
(72) Inventor: GERHARDT, Lutz Christian, Eindhoven (NL); HIWALE, Sujitkumar, Eindhoven (NL)
(74) Representative: Philips Intellectual Property & Standards

(57) **Abstract**

The subject-matter of the present disclosure relates to a computer implemented method (100) for determining an optimised efficacy of a medical treatment based on usage of a personal care appliance (402). The method comprises receiving (101) user-related data comprising a time of administration of the medical treatment, receiving (102) sensor data (t1-t4) from the personal care appliance captured during usage of the personal care appliance, and a usage time of the personal care appliance, mapping (103) the received sensor data to an efficacy model (302) corresponding to the medical treatment, based on the received user-related data and usage time of the personal care appliance, determining (104) a first treatment efficacy based on the mapping to the efficacy model, and determining (105) a second treatment efficacy based on changing the mapping by changing at least one of the usage time of the personal care appliance or the efficacy model (304, 306).

## Description

### FIELD OF THE INVENTION

The subject-matter of the present disclosure relates to a method of determining efficacy of (and/or adherence to) a medical treatment; in particular, a method of determining efficacy of (and/or adherence to) a medical treatment based on usage of a personal care appliance. Yet more specifically, the method may be a computer implemented method.

### BACKGROUND OF THE INVENTION

Incorrect administration of a medical treatment - e.g., due to incorrect regularity, incorrect dosage, etc - has a negative impact not only on (longer-term) treatment success of an underlying primary disease, but can also negatively affect other activities such as personal care routines. Many (chronic) diseases (e.g., (hand arthritis, tremor, diabetes) impact and affect a person's ability to perform a personal care routine (e.g., oral care) and require use of medication at its optimum therapeutic effect or therapeutic time window (TW) to be performed properly. For example, oral care activities require sufficient motor control, visual and cognitive coordination to ensure proper cleaning and health outcomes.

If treatment application and efficiency is not controlled, this may also promote worsening of the primary disease or foster development of co-morbidities or oral-systemic disease complications. Furthermore, monitoring treatment administration and compliance to medication is difficult in an unsupervised home setting and relies purely on a unregular personal patient-GP communication.

It is therefore now highly desirable to monitor medical treatment adherence and efficiency in a home setting with a view to enabling use of a personal care device at an optimum therapeutic effect point.

### SUMMARY OF THE INVENTION

The present invention is defined according to the independent claims. Additional features will be appreciated from the dependent claims and the description herein. Any embodiments which are described but which do not fall within the scope of the claims are to be interpreted merely as examples useful for a better understanding of the invention.

In particular, embodiments of the invention relate to enabling the use of an oral care device (e.g. powered toothbrush, flossing device, etc) at optimum therapeutic effect point or maximum therapeutic time window. Such solutions help engaging persons - especially the ones suffering from cognitive and visual coordination issues - to perform regular oral routine with high confidence level to achieve good outcome (engagement driver).

Suitably, in a first aspect of the invention, there is provided a computer implemented method for determining an optimised efficacy of a medical treatment based on usage of a personal care appliance. The method comprises receiving user-related data comprising a time of administration of the medical treatment, receiving sensor data from the personal care appliance captured during usage of the personal care appliance, and a usage time of the personal care appliance, mapping the received sensor data to an efficacy model corresponding to the medical treatment, based on the received user related data and usage time of the personal care appliance, determining a first treatment efficacy based on the mapping to the efficacy model, and determining a second treatment efficacy based on changing the mapping by changing at least one of the usage time of the personal care appliance or the efficacy model.

Suitably, determining the second efficacy may indicate parameters for improved treatment of the medical condition, or may indicate that there are no improvements available and current treatment plans are the most appropriate. In this way the present method helps engage users - especially the ones suffering from cognitive and visual coordination issues - to perform regular oral routine with high confidence level to achieve good outcome. Moreover, the method provides an unobtrusive approach to maximize medication effectiveness and use medication at its optimum therapeutic effect.

In an example, the determination of the second treatment efficacy may be performed based on determining that the first efficacy does not meet a predetermined criterion. In other words, the final step of the method is only performed when it is deemed that there may be benefit in doing so.

In an example, changing the usage time of the personal care appliance comprises changing at least one of a usage start time and a usage duration relative to the efficacy model. In this way the user may be informed of an optimal time for personal care, such as brushing teeth, while maintaining their current treatment (e.g., medication) routine.

In an example, changing the efficacy model comprises changing at least one of a time or concentration of administration of the medical treatment. Suitably, changing a time of administration of the medical treatment may comprise changing an application (e.g., intake) pattern of the medical treatment. In this way the user may maintain a preferred personal care routine and map their medicaments to their preferred routine.

In an example, changing the efficacy model may comprise changing the medical treatment. Again, the user may find it easier to maintain their personal care routine and adapt their treatment around that, possibly necessitating a change in medicine.

In an example, changing the efficacy model may comprise transmitting a control signal to the personal care appliance to activate an auxiliary treatment function of the personal care appliance. Here the method may also comprise receiving first historical sensor data corresponding to when the auxiliary treatment function is not active during usage of the personal care appliance, second historical sensor data corresponding to when the auxiliary treatment function is active during usage of the personal care appliance, and adjusting a setting of the treatment function of the personal care appliance based on analysis of the first and second historical sensor data and the mapping of the received sensor data. In this way the user of an adaptive device comprising an auxiliary treatment function (e.g., hot/cold treatment, tremor reduction) may have the usage of the auxiliary treatment function better tailored with their medical treatment to achieve maximum beneficial effect. For instance, in the case of medication to treat physical disabilities, the use of specific adaptive tremor or joint-stiffness reduction auxiliary function can help to reduce disease symptoms. However, use of such specific device with auxiliary function may not always completely eliminate such symptoms. Furthermore, the use of these devices may be more power-/energy-consuming due NIR-light LEDs treatment (i.e., requires more frequent recharging) and may exert (mechanical) strain on the motor (due to asymmetrically driving) potentially affecting oral care or toothbrush lifetime, i.e., these devices should not be continuously used, but only if needed.

In an example, the method may comprise outputting information corresponding to at least one of the first and second treatment efficacy models, including controlling a display to display a recommended change in at least one of the usage time of the personal care appliance, time of administration of the medical treatment, and the medical treatment.

In an example, the sensor data may comprise data captured by an inertial measurement unit, IMU, and mapping the received sensor data to the efficacy model comprises utilising the IMU data as a proxy parameter for a parameter in the efficacy model.

In an example, the sensor data may comprise data captured by one or more of fluid sensors, a gas sensor, a galvanic skin response sensor, an image sensor or multi-hyperspectral camera, a temperature sensor, an electrogram, a sensor configured to detect concentrations of an administered drug/medication, or a sensor configured to detect any parameter of the efficacy model.

In an example, the medical treatment may comprise a pharmacological treatment, and the efficacy model may comprise a corresponding (predetermined) pharmacometrics model. Suitably, the pharmacometrics model may comprise at least one or a combination of a pharmacokinetics curve, a pharmacodynamics curve.

In another aspect of the present invention, there is also provided an electronic apparatus comprising at least one processor configured to carry out the aforementioned method.

In another aspect of the present invention, there is also provided a transitory or non-transitory computer readable medium, having instructions stored thereon that, when executed by a processor, cause the processor to perform the aforementioned method.

These and other aspects of the present invention will be apparent from and elucidated with reference to the embodiment(s) described hereinafter.

### BRIEF DESCRIPTION OF DRAWINGS

The embodiments of the present inventions may be best understood with reference to the accompanying figures, in which:
Fig. 1 shows an example computer implemented method for determining efficacy of a medical treatment based on usage of a personal care appliance;
Fig. 2 shows an example of using a pharmacokinetics (PK) based model; Fig. 2A shows an example PK curve; Fig. 2B shows a graph of measured to expected concentration; Fig. 2C shows a ratio of measured to expected pharmacological concentration;
Fig. 3 shows an example of using a combined pharmacokinetics-pharmacodynamics (PK/PD) based model; Fig. 3A shows an example PK/PD curve; Fig. 3B shows a graph of measured medication effect; Fig. 3C shows a ratio of measured to expected pharmacological effect;
Fig. 4 shows examples of changing the efficacy model by changing a usage time of the personal care appliance;
Fig. 5 shows examples of changing the efficacy model by changing one of a time of administration of the medical treatment, a concentration of the medical treatment, a frequency of administration/intake, or the medical treatment;
Fig. 6 shows an example system for performing the methods herein;
Fig. 7 shows a more detailed method of performing the techniques herein;
Fig. 8 shows an example method involving a personal care device with auxiliary treatment function;
Fig. 9 shows an example effect on a PK/PD curve from an auxiliary treatment function.

### DESCRIPTION OF EMBODIMENTS

Personal care activities, such as oral care, require sufficient motor control, visual and cognitive coordination to ensure proper cleaning and health outcomes. However, medication or lack of medication can affect these. For example, many (chronic) diseases impact and affect a person's ability to perform proper oral care routine - e.g. along recommended instructions for use or recommendations of dentists - and require regular use of medication to alleviate disease symptoms.

For example, joint disorders (inflammatory/osteo arthritis) or motor disabilities (e.g., tremors, joint stiffness/rigor) can adversely affect coordination activities and thus quality of oral hygiene. Such persons are prescribed medications to alleviate joint pain/stiffness. As another example, cognitive disorders or depression can lead to the result a person does not or only brush randomly for a short time < 2 mins.

Table 1 summarizes a list of relevant (chronic) diseases affecting physical motor, cognitive and visual coordination, the typical treatment for each disease, and their potential (oral) side effects.

**Table 1:**

| Use case: Chronic disease | Worldwide prevalence [%] | Treatment (examples) | Primary therapeutic effect | (Oral) side effects caused by medication |
|---|---|---|---|---|
| Hand/Finger arthritis incl joint stiffness | ≈40-50 % | Anti-inflammatory agent, painkiller | Relieves inflammation and pain | Dry mouth, gum swelling |
| Bruxism and Temporo-mandibular joint disorder (TMJD) | ≈25 % | Anti-inflammatory agent, painkiller | Relieves inflammation and pain | Dry mouth, gum swelling |
| Non-essential and Parkinson tremors | ≈ 1- 5% | β-blocker, Anti-seizure medicines, Tranquilizers | Reduce hand shaking and joint stiffness | Dry mouth, blurred, vision, Fatigue, anxiety |
| Depression | ≈ 5 % | Antidepressants | Relieve from stress, anxiety | Dry mouth, acidic tooth decay |
| Diabetes mellitus | ≈ 9 % (1 of 11) | Insulin | Sugar production lowering/inhibitio n agents | Blurred vision |
| COPD/Asthma | 6-12 % | Bronchodilators, oxygen therapy | Makes breathing easier by relaxing/widening airways | Dry mouth, Hand trembling |
| Cognitive disorders incl. mild dementia | 19 % | Mental function enhancers (Nootropics) | Improve state of mind | Dry mouth, daytime sleepiness, blurry vision |
| Hypertension | 28-34 % | ACE inhibitors, diuretics Angiotensin-2 receptor blockers | Lower bood pressure | Dry mouth, gum swelling and overgrowth |

In order to better engage persons - especially the ones on-medicine to treat physical, cognitive and visual coordination issues - to perform regular personal care routine with high confidence level, there is a need for solutions to maximize medication effectiveness and use medication at its optimum therapeutic effect point or time window (TW).

Accordingly, medicine-taking persons want to know (1) how to perform oral hygiene at their preferred oral care time / time window (with least expected complications) or (2) obtain guidance on an optimum routine time window where therapeutic effect is largest and/or treatment (e.g., drug) side effect the lowest. Moreover, those with specific adaptive devices to reduce/counteract hand related dexterity issues may not completely eliminate symptoms of tremor and joint stiffness. Therefore, it is highly beneficial to determine optimum care (e.g., teeth brushing) time to further augment the therapeutic effect and exploit the usability of such devices towards maximum effectiveness.

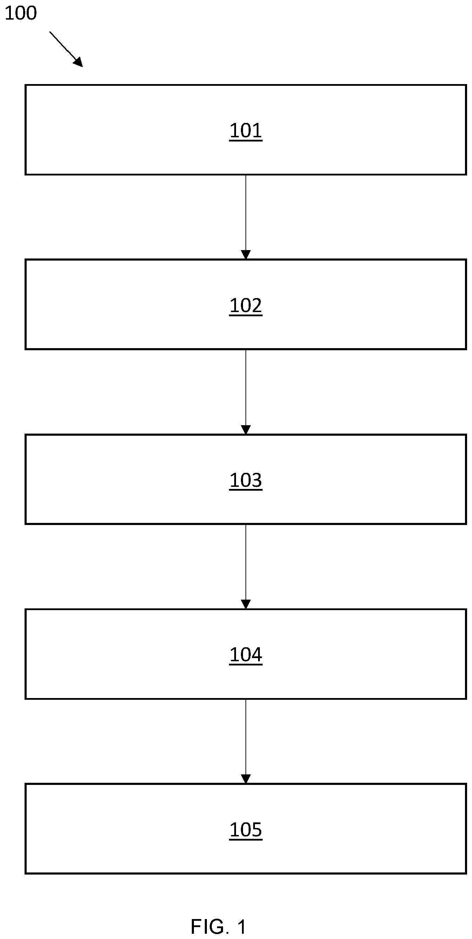

Suitably, Fig. 1 shows an example computer implemented method 100 for determining efficacy of a medical treatment, based on usage of a personal care appliance, in order to determine an optimum care routine.

At step 101, the method comprises receiving user-related data comprising time of administration of a medical treatment. The user-related data may more generally be considered as medical data. Suitably, the user-related data may comprise information of what the medical treatment is (e.g., a type of drug), administration time of the medical treatment (including actual time of administration and e.g., advised times and/or frequency of application), and also typical schedule for usage of the personal care appliance (e.g., approximate start time, duration, etc), and so on. An example drug usage intake schedule might indicate that medication is taken twice a day (morning and evening), or three times a day (morning, midday, evening). An example personal care appliance schedule might indicate that the personal care routine is performed in the morning and evening (e.g., oral hygiene) or just one or the other (e.g., shaving); it should go without saying that the received data should be relevant to the type of personal care appliance and the usage thereof to which the method is being based.

Suitably, the user-related data may comprise a first time corresponding to a first administration of the medical treatment for a predetermined time period (e.g., over a day), and a second time corresponding to a subsequent administration of the medical treatment for the predetermined time period (e.g., when a second administration during a day occurs).

In this way, the start time of the personal care routine can be used (later in the method) as a well-defined reference time point to time-correlate / sync medication efficacy: for example, medical treatment intake curves and/or medical effect curves, as discussed in more detail below.

At step 102, the method comprises receiving sensor data from the personal care appliance. The sensor data is captured during usage of the personal care appliance so that the captured data may be indicative of a primary therapeutic effect of a medication or a side effect of the medical treatment: for example, the sensor data may be analysed to determine a degree of severity of one or more of the side effects listed in Table 1. Suitably, a usage time of the personal care appliance is also received; preferably the usage time includes at least a start time, but may also include a duration and end time.

In one example, the sensor data may comprise IMU data captured by an inertial measurement unit (which may be provided on the personal care appliance). In another example, the sensor data may comprise data captured by one or more of a fluid sensor, a galvanic skin response sensor, an image sensor or multi-hyperspectral camera, a temperature sensor, and an electrogram, any (chemical) sensor to detect concentrations of an administered drug/medication. The sensor data may comprise IMU data and any other sensor data just listed. It will of course be appreciated that there may be other examples of sensors and their corresponding data, not listed here, which are relevant to the determining of treatment efficacy that could be utilised within the present method.

At step 103, the method comprises mapping the sensor data received at step 102 to an efficacy model corresponding to the medical treatment. The mapping is based on the user related data received at step 101 and the usage time of the personal care appliance received at step 102.

Here the efficacy model may be any suitable model which indicates a person's response to a medical treatment over time. In preferred examples, whereby the medical treatment is pharmacological (i.e., drug based), the efficacy model may be a pharmacometrics model, such as a pharmacokinetics (PK) curve, a pharmacodynamics curve (PD), or a combination thereof (PK/PD). Pharmacokinetic models describe how the body reacts to a drug in terms of absorption, distribution, metabolism, and excretion (ADME). Pharmacodynamic models describe how a drug affects the body by linking the drug concentration to an efficacy, therapeutic effect (or safety) metric. It will be appreciated that the pharmacometrics model will be predetermined based on the medical treatment the user is applying. It will also be appreciated that other clinically validated efficacy models may also be utilised within the present technique.

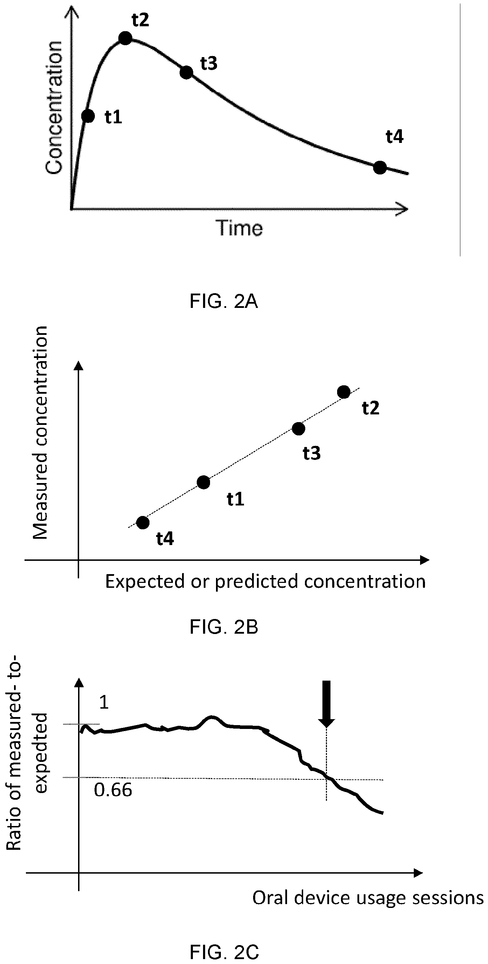

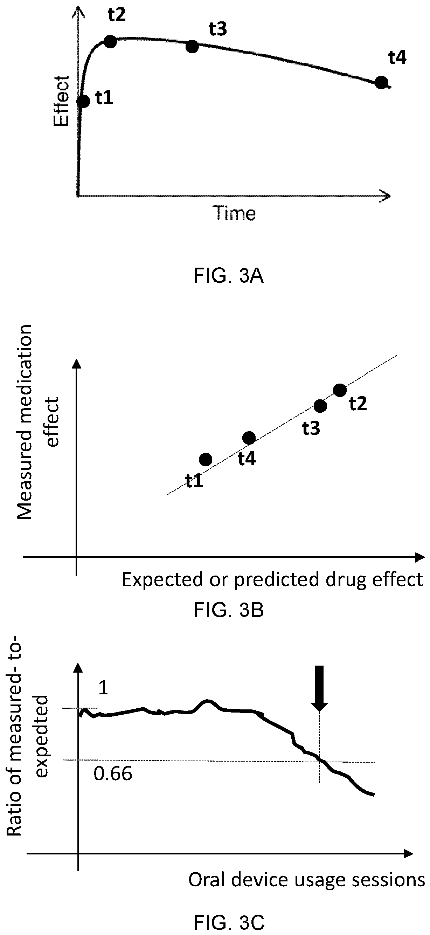

Suitably, in the case of PK, PD, and PK/PD curves, the mapping may comprise mapping the personal care appliance start time and/or duration and/or stop time onto the PK, PD, PK/PD curves. Fig. 2A shows an example of such a mapping to a PK curve at various times (t1-t4) during usage of an appliance. Fig. 3A shows a similar example for mapping to a combined PK/PD curve at various times (t1-t4) during usage of an appliance.

From the curves can be derived graphs for expected (or predicted/forecasted/predetermined) vs actual medication effect, as shown by Figs. 2B and 3B (following from the mapping in Figs. 2A and 3A respectively). The mapping may also comprise determining at least one of a ratio of measured to expected pharmacological concentration, as in Fig. 2C, or a ratio of measured to expected pharmacological effect, as in Fig. 3C (following from Figs. 2B and 2C respectively).

At step 104, the method comprises determining a first efficacy of the medical treatment based on the mapping. Suitably determining treatment efficacy may comprise determining a metric that the first efficacy does (or does not) meet a predetermined criterion. For example, in the case of pharmacological treatment, determining that at least one of a treatment effect (Fig. 3) or a treatment concentration (Fig. 2) satisfies a threshold value. In one example, the predetermined criterion is that the determined first efficacy, which may be value derived from the mapping, is 90% of a target of a target value.

At step 105, the method comprises determining a second treatment efficacy based on changing the mapping. In particular, changing the mapping comprises changing at least one of the usage time of the personal care appliance or and the efficacy model. Put another way, the first treatment efficacy may be determined based on a first mapping (that is, the mapping described above may be considered the first mapping), and the method performs a second mapping based on a simulated or forecasted change to at least one of the usage time of the personal care appliance or and the efficacy model.

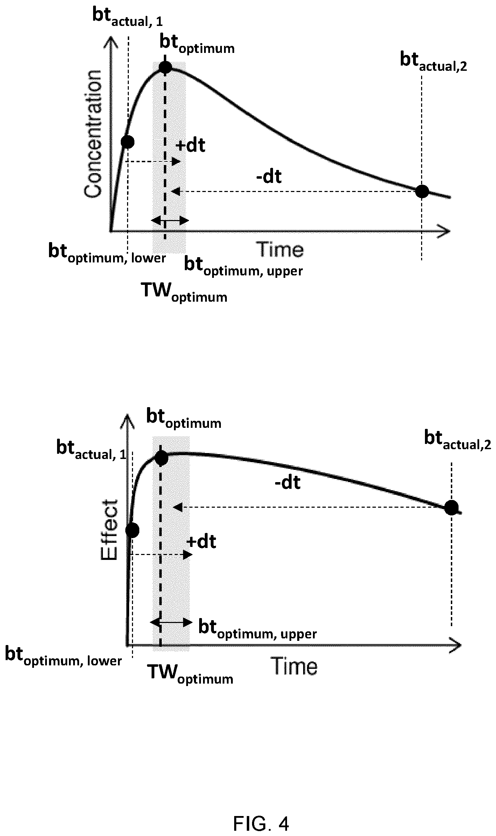

Changing the usage time of the personal care appliance may comprise changing at least one of a usage start time and a usage duration relative to the efficacy model, as shown by Fig. 4 (top shows PK modelling, bottom shows PK/PD modelling). That is, the method may determine an optimum time for the user to perform the oral care routine (such as brushing teeth), while keeping the same efficacy model that was used for the first mapping (i.e., same medication, same intake time or frequency, etc). Put another way, the second mapping is performed based on mapping a simulated or forecasted change in the usage time (start or duration) of the personal care appliance relative to the (existing) efficacy model, and a second efficacy determined therefrom.

For example, in the case of oral care, a fixed medication schedule can be used as the reference framework (i.e., the efficacy mode), and an optimum oral care routine window (TWₒₚₜᵢₘᵤₘ) with lower bound (bt_{optimum, lower}) and upper bound (bt_{optimum, upper}) or optimum brushing time point (btₒₚₜᵢₘᵤₘ) in an iterative approach by comparing actual with optimum medication effect or a proxy parameter thereof (e.g. tremor signal picked up by IMU, hand grip patterns measured by IMU). Based on the comparison, the user can be encouraged to shift actual brushing times (bt_{actual,1} bt_{actual,2}) by a time difference +dt or -dt towards optimum time btₒₚₜᵢₘᵤₘ along the PK or PK-PD curve.

Moreover, additional data may be used in the proposing a changed mapping (and the determined efficacy therefrom), which may be received as part of the user-related data at step 101. For example, if btₒₚₜᵢₘᵤₘ coincides with breakfast or dinner time, then to allow for at least 30 mins recovery time of the oral pH after food consumption to avoid excessive tooth wear, the upper bound of the time window may be adjusted.

Changing the efficacy model may involve changing one of a time of administration of the medical treatment, a concentration of the medical treatment, a frequency of administration/intake (or application pattern), and the medical treatment itself. That is, the method may determine an optimum efficacy model in which the personal care routine is performed at a predetermined time; the predetermined time being set by the captured sensor data (step 102) and mapping thereof to the (initial) efficacy model, or by receiving a preferred time along with the user related data of step 101. Put another way, the second mapping is performed based on mapping a simulated or forecasted change in the efficacy model (by time or concentration) relative to the usage time of the personal care appliance, and a second efficacy determined therefrom.

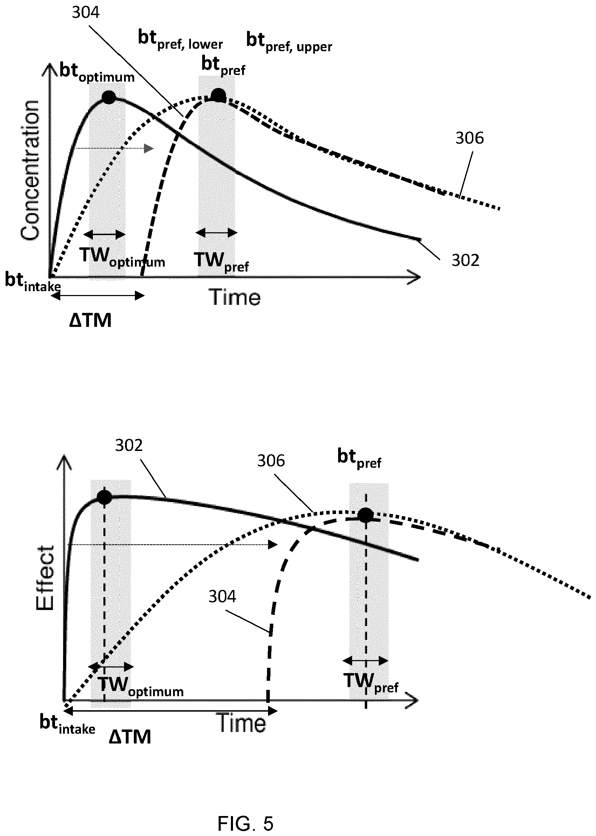

For example, as shown in Fig. 5 (top shows PK modelling, bottom shows PK/PD modelling), curve 302 shows the first efficacy model (step 103) to which the sensor data is mapped, optionally indicating an optimum time for the personal care routine (similar to Fig 4) based on the received sensor data. Curve 304 shows an example where the efficacy model is for the same treatment as currently being taken, and with the same frequency and/or (as indicated by curve 304 having generally the same shape as curve 302), but the administration time has been shifted in order for the optimum point on the curve to correlate to the preferred (i.e., predetermined) usage time of the personal care appliance. Curve 306 shows an example whereby one or more of drug concentration, frequency of intake, or the medical treatment itself (e.g., drug) have been changed while maintaining the same intake time.

Suitably, in an example for oral care, pharmacometrics (PK, PK-PD) curves/characteristics may be dynamically changed (or adapted) in order to direct optimum therapeutic response to a preferred oral care time point or time window; the time window being indicated by the user or medical practitioner, For example, a fixed preferred brushing time (bt_{pref}) or preferred brushing time window (TW_{pref}) with lower and upper bound bt_{pref, lower}; bt_{pref, upper}) may be set, from which may be determined suitable medication curves (btₒₚₜᵢₘᵤₘ, TWₒₚₜᵢₘᵤₘ, btᵢₙₜₐₖₑ) which indicate a shift in medication intake ΔTM or adaptation of PK, PK-PD such that maximum medication effect/concentration coincides with and occurs at preferred brushing time.

Suitably, comparing the preferred with ideal/optimum medication effect and shift medication intake time/curve (PK, PK-PK curve towards the pre-determined and preferred "fixed" brushing time) allows for iterative improvements in PK-PC curve progression characteristics in such a way the optimum goes through the preferred brushing time window or preferred brushing time.

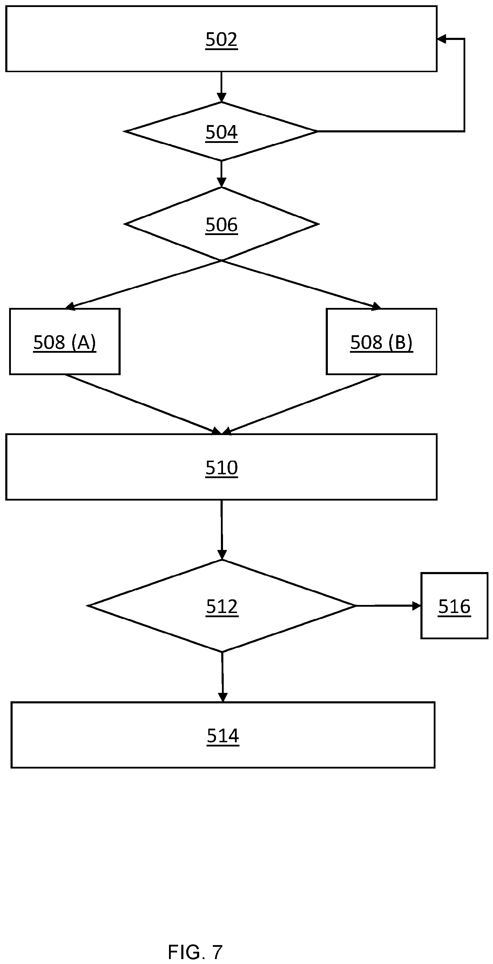

For example in the third embodiment, we get a fixed preferred brushing time (btpref) or preferred brushing time window (TWpref) with lower and upper bound btpref, lower; btpref, upper) and determine from known medication curves (btoptimum, TWoptimum, btintake) and patient data the required shift in medication intake ΔTM or adaptation of PK, PK-PD such that maximum medication effect/concentration coincides with and occurs at preferred brushing time (Fig. 7).

It should be appreciated that, in some examples step 105 is only performed after checking that the first efficacy does not meet the predetermined criteria, with the aim of determining the second treatment efficacy being performed in order to find an improved efficacy which satisfies the predetermined criteria upon which the first efficacy is assessed; for example, the second efficacy is greater than 90% of a target value. Suitably, step 105 may be considered to involve an iterative process, the (second) mapping is changed a number of times until the assessment criteria is determined to converge to an optimum; for example, an optimum value of concentration and/or effect (or variations in those parameters over time).

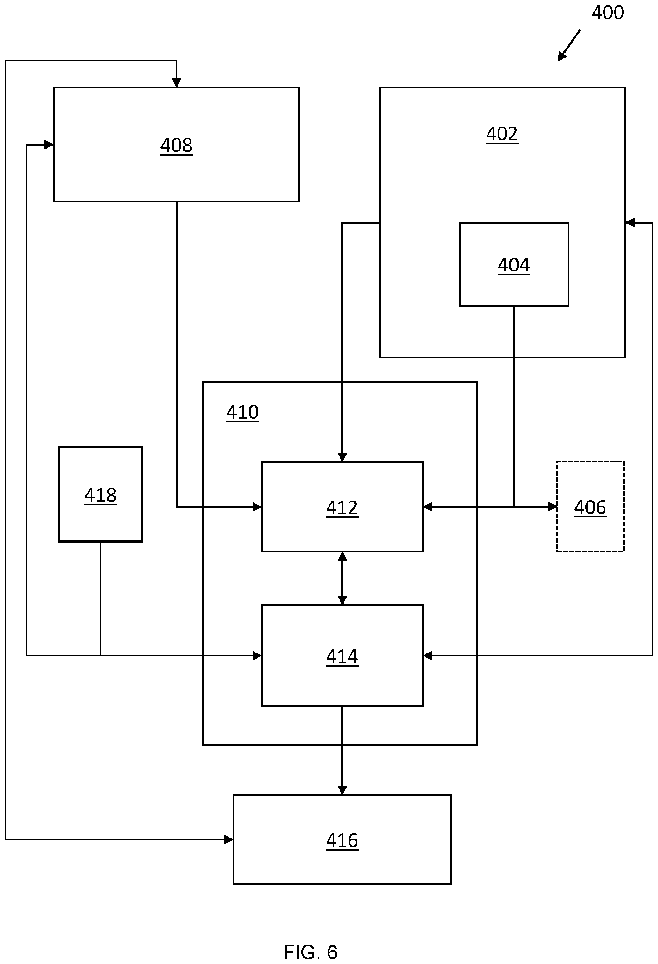

Fig. 6 shows an example system 400 configured to apply the techniques herein in the case of an oral hygiene device 402, while Fig. 7 shows the method of Fig. 1 in more detail as applied to the system 400.

The system comprises the oral device 402, which has on-board sensors 404, optional external sensors 406 (that is, external to the oral device 402), a server 408, an electronic apparatus 410 comprising a processor 412 (e.g. for executing an algorithm) and a data communication module 414, a user interface (UI) 416 (integrated in 402 or remote), which may be coupled to the electronic apparatus 410 and/or server 408, and optionally an automatic drug dispenser 418.

Referring now to Fig. 7: At step 502, the method comprises initiating a medication mode in the oral device 402 or in/via a remote accessory such as smart phone App. Initiation may be via input direct to the oral device 402 (e.g., by a button), or may be achieved by suitable input to the apparatus 410 which communicates an activation signal to the oral device 402 via the communication module 414. Initiating the medical mode may cause the activation of a suitable application, or medication program, on the apparatus 410. For instance, specific hardware and software user interfaces can be used to initiate and verify initiation of a medication intake and compliance mode. Once the initiation is successfully verified, the user obtains feedback on the device or Sonicare App. Hardware may include, for example, a toothbrush handle with medication mode or display showing medication icon, while software may include an App with activated medication compliance measurement mode (e.g. add a new tab or activate a greyed-out window).

At step 504, the method comprises validating initiation of the medical mode by the processor 412. If yes (i.e. successfully initiated), the method proceeds to step 506; if no (initiation failed), the system may return to step 502. In an example, step 504 may be considered a check that a suitable app on the electronic apparatus is running and functioning correctly, and if no, the processor waits for the app to function before continuing.

At step 506, the processor receives the user related data comprising time of administration of the medical treatment, as at step 101 (and, of course, the type of medical treatment). The user-related data is received from server 408 (via the communication module 414), being stored, for example, in a suitable database accessible by the user via appropriate authentication, etc. The time of administration received from the database may be updated manually by the user, e.g., via an app on the electronic apparatus 410, or may be obtained automatically, for example from a time of access of automatic drug dispenser 418.

Moreover, the database may comprise, for example, a list of types and number of medications taken by the user, frequency of administration, time schedule, PD-PK model or PD concentration curves of medication, individual specific properties of patient such as age, height, weight, severity of diseases, eating habits, EMR (Electronic Medical Record) data of patient, and so on.

Based on the received user-related data, step 506 also comprises determining (by the processor 412), whether the primary underlying disease affect physicals, cognitive or visual coordination, and then determines a type of sensor data (direct data, proxy parameters, or both) used to determine medication efficacy (as at step 512). At step 508, the relevant sensor data is received by the processor 412 via the communication module 414.

In a preferred example, characteristic signatures of proxy parameters from sensors 404 common to many oral devices may be utilised (step 508A). For example, in order to analyse pain or inflammation relief medication to tackle painful mouth opening or impaired grip due to arthritis/TMJ, it is possible to analyse IMU/drive train signatures to determine finger/hand grips related to joint stiffness, mouth opening or brushing patterns to provide feedback on drug intake and effectiveness. Anti-tremor medication intake and effectiveness may be assessed by measuring hand tremor signature picked up by IMU or microphone in oral device. Regular intake/effect of anti-depressants may be assessed by determining that a full brushing session was completed or whether brushing is being systematically omitted.

In other examples direct measurement of drug response (therapeutic effect) may be determined (step 508B) by measurements using any (additional) sensor 404 or measurement in oral device or even external sensor 406 (e.g. part of a healthwatch, or smart phone). For example, the direct measurement could include measurement of concentration of (saliva) biomarkers incl. pH, bad breath gas sensing concentration, galvanic skin response, electrodermal activity, vital signs (blood oxygen, respiration rate, body core temp, heart rate, blood pressure), or image-based measurements e.g. from hyperspectral images incl color using camera's or image sensors.

Table 2 shows sensor data indicative for symptoms related to (chronic) diseases, not limited to but including physical, cognitive and visual coordination.

**Table 2:**

| Use case: Chronic disease | Oral (device) proxy signature or direct measurement parameter indicative for drug effectiveness | Medication /drug |
|---|---|---|
| Hand/Finger arthritis incl joint stiffness | Grip pattern, Brushing pattern | Anti-inflammatory agent, painkiller such as ibuprofen |
| Bruxism and Temporo-mandibular j oint disorder (TMJD) | Mouth opening angle determined from IMU, GSR | Anti-inflammatory agent, painkiller such as ibuprofen |
| Non-essential and Parkinson tremors | Low frequency vibration signature on Sonic frequency, grip pattern | β-blocker, Anti-seizure medicines, Tranquilizers |
| Depression | Brushing duration pattern (length, regularity/consistency) | Antidepressants |
| Diabetes mellitus | Glucose level in saliva alone or in combination with brushing pattern | Insulin |
| COPD/Asthma | Brushing duration pattern (length, regularity/consistency), saturation | Bronchodilators, oxygen therapy |
| Cognitive disorders incl. mild dementia | Brushing duration pattern (length, regularity/consistency) - compliance | Mental function enhancers (Nootropics) |
| Hypertension | Cholesterol biomarker in saliva, PPG signatures to estimate blood pressure | ACE inhibitors, diuretics Angiotensin-2 receptor blocker |

At step 510, the oral device usage time window is mapped onto a predetermined pharmacometrics curve accessible from the database 408 and metrics for drug intake compliance and drug effectiveness are determined. That is, the first mapping to the sensor data is performed.

At step 512, the processor 412 determines the efficacy of the treatment. If it is determined that the current treatment regimen is effective (e.g., by virtue of the determined first efficacy meeting the predetermined criteria), then at step 516 the user may be suitably informed as such via user interface 416 to provide reassurance to the user. Likewise, data indicative of the effectiveness may also be uploaded to the server 408 to update the medical database. In other words, the result of the determining the efficacy of the medical treatment may be transmitted to the server 408. If the current treatment is determined to not be effective (e.g., by the first efficacy being below the predetermined criteria), then the method proceeds to step 514.

At step 514, the second efficacy is determined as described above. Suitably, the changed efficacy model may be obtained from the server 408 (e.g., by requesting a model with a different treatment intake pattern or concentration). Suitably, by using the received time-correlated sensor data and pharmacometrics model data, actual vs optimum effect (ΔE), time difference dt, concentration difference Δc, slope of effect difference or rate of change of effect time (ΔE/dt) or (Δc/dt), may be compared. Based on the comparison and calculated efficacy parameter, a potential change, or adaptation of, either oral device usage time (i.e., time shift) or pharmacometrics model characteristics (e.g. time shift of medication intake and/or use of different medication), may be determined for achieving optimum therapeutic drug effect and maximizing medication effectiveness (drug response) for improved oral care routine.

Once a suitably optimal second efficacy is determined, feedback may be provided to the user via the UI 416, and also transmitted to the server 408 (and flagged for review by e.g., a medical practitioner). In this way may the method recommending a change in at least one of the parameters of the efficacy model(s) - usage time of the personal care appliance, administration time/frequency of the medical treatment, and medical treatment - so as to recommend a change in treatment of the user.

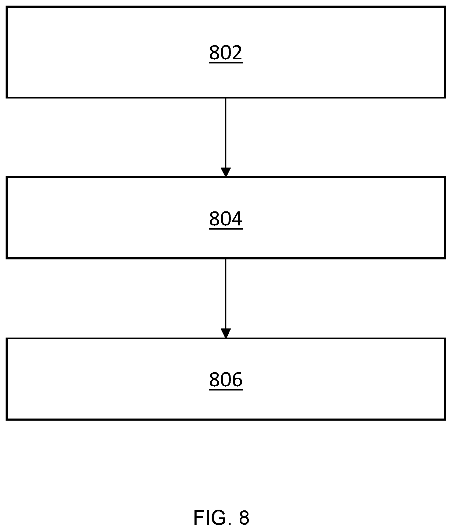

Fig. 8 shows a modification of the above techniques in which an adaptive personal care device with oral device with an auxiliary treatment function - such as a tremor reduction or join stiffness treatment function - may be used to further optimise the second treatment efficacy as use of such specific devices with auxiliary function alone without optimising second treatment efficacy may not always completely eliminate such symptoms of tremor and joint stiffness. However, the use of these devices with auxiliary function is more power-/energy-consuming due NIR-light LEDs treatment (i.e., requires more frequent recharging) and exerts (mechanical) strain on the motor (due to asymmetrically driving) potentially affecting toothbrush lifetime, i.e., these devices should not be continuously used, but only if needed. Therefore, it may be desirable to selectively activate the auxiliary function.

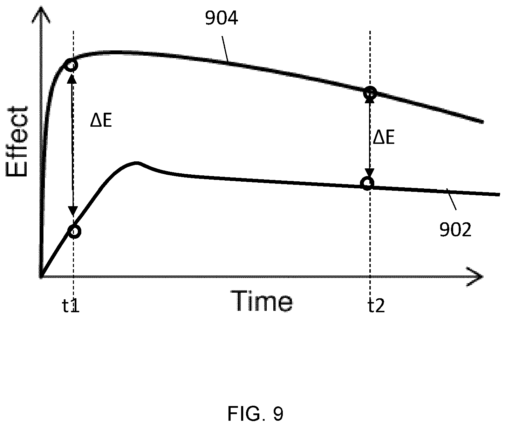

Suitably, in this example, known calibration curves or the relationship between device usage time and medication effect (PK-PD curves, or proxy parameter) may be compared for a first state in which an auxiliary treatment function is "on" (e.g., with tremor/heat treatment function active) and a second state in which the auxiliary treatment function is "off" (i.e., without auxiliary treatment) in order to (selectively) probe the effect of the auxiliary treatment function on the efficacy model (and determined second efficacy) as part of determining optimum medication effect at a preferred care time or determination of optimum care time based on preferred medication routine. Fig. 9 shows an example of the effect of an auxiliary treatment function (i.e., an adaptive device) on an example PK/PD curve: curve 902 shows the adaptive device "off"; curve 904 shows the adaptive device "on".

Suitably, at step 802, step 105 of Fig. 1 may be modified to, as part of changing the efficacy model, receive first historical sensor data (either from the care device or the server) corresponding to when the auxiliary treatment function is not active, and second historical sensor data when the secondary or auxiliary treatment function is active (and the personal care appliance is in use) That is, the historical sensor data may be considered to correspond to normal usage of the personal care device.

At step 804, the method comprises analysing both of the first and second historical sensor data to compare the effect of the auxiliary treatment function on the efficacy. That is, step 804 may comprise changing the efficacy model based on changing the activation state of the auxiliary treatment function, and map the received sensor data to the changed efficacy model in determination of the second efficacy.

At step 806, the method comprises adjusting a setting of the auxiliary treatment function based on the analysis of the first and second historical sensor data and the mapping thereof to the received sensor data. Suitably, step 806 may comprise transmitting a control signal to the personal care device to activate the auxiliary treatment function of the personal care appliance.

Suitably, in the example of Fig. 8, activation of the auxiliary treatment function may further enhance/augment the therapeutic effect and usability of the device at the time point of maximum medication effectiveness (and exploit the usability of such devices towards maximum effectiveness). It will be appreciated that even if an adaptive oral device is used to reduce/counteract hand related dexterity issues is used, this may not completely eliminate symptoms of tremor and joint stiffness. As such, the present techniques allow for improved usage of an adaptive device by mapping the usage of the device, and in particular the auxiliary treatment function, to the optimum therapeutic window.

In this way, auxiliary treatment modes such as heat/cold treatment and tremor reduction may be activated at optimum times, frequency, etc, based on the actual medication effect and the type of arthritis (inflammatory/osteo arthritis) and physical disability/limitation during brushing. In other words, by knowing where a user of an adaptive device is on their medication curve, the present techniques can optimise their device usage for maximised medication effectiveness and improved oral care.

In summary then, example embodiments of a method for determining efficacy of a medical treatment based on usage of a personal care appliance, with a view to optimising a care routine, have been described.

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements. Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. A computer implemented method (100) for determining efficacy of a medical treatment based on usage of a personal care appliance (402), comprising:
receiving (101) user-related data comprising a time of administration of the medical treatment;
receiving (102) sensor data (t1-t4) from the personal care appliance captured during usage of the personal care appliance, and a usage time of the personal care appliance;
mapping (103) the received sensor data to an efficacy model (302) corresponding to the medical treatment, based on the received user-related data and usage time of the personal care appliance;
determining (104) a first treatment efficacy based on the mapping to the efficacy model; and
determining (105) a second treatment efficacy based on changing the mapping by changing at least one of the usage time of the personal care appliance or the efficacy model (304, 306).

2. The method of claim 1, wherein the step of determining the second treatment efficacy is performed based on determining that the first efficacy does not meet a predetermined criterion.

3. The method of claim 1 or 2, wherein changing the usage time of the personal care appliance comprises changing at least one of a usage start time and a usage duration relative to the efficacy model.

4. The method of any preceding claim, wherein changing the efficacy model comprises changing at least one of a time or concentration of administration of the medical treatment.

5. The method of claim 4, wherein changing the time of administration of the medical treatment comprises changing an application pattern of the medical treatment.

6. The method of any preceding claim, wherein changing the efficacy model comprises changing the medical treatment.

7. The method of any preceding claim, wherein changing the efficacy model comprises transmitting a control signal to the personal care appliance to activate an auxiliary treatment function of the personal care appliance.

8. The method of claim 7, wherein transmitting the control signal to the personal care appliance comprises:
receiving (802) first historical sensor data corresponding to when the auxiliary treatment function is not active during usage of the personal care appliance, second historical sensor data corresponding to when the auxiliary treatment function is active during usage of the personal care appliance, and
adjusting (806) a setting of the treatment function of the personal care appliance based on analysis (804) of the first and second historical sensor data and the mapping of the received sensor data.

9. The method of any preceding claim, further comprising outputting (514, 516) information corresponding to at least one of the first and second treatment efficacy models.

10. The method of claim 9, wherein outputting information corresponding to the second treatment efficacy model comprises controlling a display (416) to display a recommended change in at least one of the usage time of the personal care appliance, time of administration of the medical treatment, and the medical treatment.

11. The method of any preceding claim, wherein the sensor data comprises data captured by an inertial measurement unit, IMU, and mapping the received sensor data to the efficacy model comprises utilising the IMU data as a proxy parameter for a parameter in the efficacy model.

12. The method of any preceding claim, wherein the sensor data comprises data captured by one or more of fluid sensor, a gas sensor, a galvanic skin response sensor, an image sensor or multi-hyperspectral camera, a temperature sensor, an electrogram, a sensor configured to detect concentrations of an administered drug/medication, or a sensor configured to detect any parameter of the efficacy model.

13. The method of any preceding claim, wherein the medical treatment comprises a pharmacological treatment, and the efficacy model comprises a predetermined pharmacometrics model.

14. The method of claim 13, wherein the predetermined pharmacometrics model comprises at least one or a combination of a pharmacokinetics curve and a pharmacodynamics curve.

15. An electronic apparatus (410) comprising at least one processor configured to carry out the method of any of claims 1 to 14.
